# EUROPEAN PATENT SPECIFICATION

(11) **EP 3 831 217 B1**
(45) Date of publication and mention of the grant of the patent: **19.02.2025**
(21) Application number: 20211734.7
(22) Date of filing: 04.12.2020
(51) Int. Cl.: A23L 33/105, A23L 33/16, A61K 36/736, A61K 36/81, A61K 36/63, A61K 33/00, A61K 31/01, A61K 31/192, A61K 31/7048, A61K 33/24, A61P 5/00

(54) **FOOD SUPPLEMENT FOR PROMOTING THE REDUCTION OF POSTPRANDIAL GLYCAEMIA**
NAHRUNGSERGÄNZUNGSMITTEL ZUR UNTERSTÜTZUNG DER REDUZIERUNG DER POSTPRANDIALEN GLYKÄMIE
COMPLÉMENT ALIMENTAIRE POUR FAVORISER LA RÉDUCTION DE LA GLYCÉMIE POSTPRANDIALE

(30) Priority: 06.12.2019 IT 201900023151
(43) Date of publication of application: 09.06.2021
(73) Proprietor: NGN Healthcare - New Generation Nutraceuticals S.r.l., 83013 Mercogliano (IT)
(72) Inventor: NOVELLINO, Ettore, 83100 Avellino (IT); TENORE, Gian Carlo, 80128 Napoli (IT)
(74) Representative: Valenza, Silvia

(56) References cited:
- CN-A- 107 648 349
- JP-A- H09 103 293
- US-A1- 2019 280 799
- ANONYMUS: "WE ARE AHT | Assorted Freeze-dried Fruits & Vegetables", 22 October 2019 (2019-10-22), XP055712452, Retrieved from the Internet <URL:https://web.archive.org/web/20191022134715/http://www.ahtfoods.com/freeze-dried/> [retrieved on 20200707]
- ELENA ZOCCHI ET AL: "Abscisic Acid: A Novel Nutraceutical for Glycemic Control", FRONTIERS IN NUTRITION, vol. 4, 13 June 2017 (2017-06-13), XP055553690, DOI: 10.3389/fnut.2017.00024
- AL-AZZAWIE H F ET AL: "Hypoglycemic and antioxidant effect of oleuropein in alloxan-diabetic rabbits", LIFE SCIENCES, PERGAMON PRESS, OXFORD, GB, vol. 78, no. 12, 16 February 2006 (2006-02-16), pages 1371 - 1377, XP028050541, ISSN: 0024-3205, [retrieved on 20060216], DOI: 10.1016/J.LFS.2005.07.029
- VESILE DUZGUNER ET AL: "Effect of Lycopene Administration on Plasma Glucose, Oxidative Stress and Body Weight in Streptozotocin Diabetic Rats", JOURNAL OF APPLIED ANIMAL RESEARCH, vol. 33, no. 1, 1 March 2008 (2008-03-01), IN, pages 17 - 20, XP055712256, ISSN: 0971-2119, DOI: 10.1080/09712119.2008.9706888
- HAMIT USLU ET AL: "EFFECTS OF CHROMIUM PICOLINATE ON OXIDATIVE STRESS AND HYPERGLYCEMIA IN EXPERIMENTAL TYPE 2 DIABETIC RATS", ASIAN JOURNAL OF PHARMACEUTICAL AND CLINICAL RESEARCH : AJPCR, vol. 11, no. 10, 7 October 2018 (2018-10-07), IN, pages 532, XP055712261, ISSN: 0974-2441, DOI: 10.22159/ajpcr.2018.v11i10.28608
- GURI ET AL: "Dietary abscisic acid ameliorates glucose tolerance and obesity-related inflammation in db/db mice fed high-fat diets", CLINICAL NUTRITION, CHURCHILL LIVINGSTONE, LONDON, GB, vol. 26, no. 1, 3 February 2007 (2007-02-03), pages 107 - 116, XP005866206, ISSN: 0261-5614, DOI: 10.1016/J.CLNU.2006.07.008
- IBRAHIM KAHRAMANOGLU ET AL: "Value-adding to the Thinned "Waste" Fruits of Pomegranates: Authentic Spoon Sweet Production Weed Survey View project Phytoremediation project View project", INDIAN JOURNAL OF HORTICULTURE, 1 January 2017 (2017-01-01), pages 208 - 211, XP055712448, Retrieved from the Internet <URL:https://www.researchgate.net/publication/323683377_Value-adding_to_the_Thinned_Waste_Fruits_of_Pomegranates_Authentic_Spoon_Sweet_Production> [retrieved on 20200707]
- F.G. DENNIS ET AL: "The history of fruit thinning", PLANT GROWTH REGULATION, vol. 31, no. 1/2, 1 January 2000 (2000-01-01), pages 1 - 16, XP055047728, ISSN: 0167-6903, DOI: 10.1023/A:1006330009160

## Description

### FIELD OF THE INVENTION

The present invention relates to the field of food supplements, in particular food supplements adapted to promote the reduction of postprandial glycaemia.

### BACKGROUND

Abscisic acid (ABA) is a plant hormone commonly known to inhibit germination. ABA, also known as dormin, plays an important role in the induction and maintenance of the dormancy of seeds, an effect largely attributed to its antimitogenic effect, which contrasts that of the hormones that stimulate growth, such as gibberellins. The inhibitory potential of ABA on the germination of seeds increases plants' tolerance to abiotic stress (e.g. temperature variations, exposure to light, osmotic stress and uptake of water and nutrients). In particular, ABA is also an important regulator of the ripening of fruits. Its concentration in fruits increases as the ripening process proceeds, until reaching a maximum level at a stage in which the fruit is still unripe, then decreasing gradually as the ripening proceeds, and almost disappearing from the fruit at the time of harvesting. Such trend responds to the needs of the fruit to conserve, during a state of dormancy, and therefore not proliferation, the cells that still do not have any protection by antioxidants that the fruit is still not able to produce. Where it is finally possible to synthesize polyphenols and carotenoids, the fruit no longer needs the presence of ABA, and its induction to dormancy, such that the concentration of such hormone is reduced, until almost disappearing once the fruit is fully ripe. From this point of view, unripe fruits are an important source of ABA. In the agronomic field, the practice of "thinning" is often adopted, a technique that consists of making up to 80% of the unripe fruits fall from the tree, so that the surviving fruits can reach the maximum maturity of their organoleptic characteristics (size, colour, flavour). The unripe fruits from the thinning process, defined as "immature fruits", therefore represent a significant volume of waste in the agri-food industry. The immature fruits from the thinning process are unripe fruits belonging to the following categories: apples, peaches, apricots.

It has recently been demonstrated that animals, including humans, are also able to produce ABA, an endogenous hormone, which would be appointed to control glycaemia, through the regulation of plasma glucose uptake by GLUT type cell receptors, with insulin saving. Clinical trials demonstrate that type 2 diabetics produce poor quantities of ABA after the intake of glucose, a clear sign that ABA acts in humans as an important glucose homeostasis regulator.

Oleuropein is a typical polyolefin constituent of extra-virgin olive oil (EVOO), even if its presence has also been discovered in other portions of the olive plant, especially the leaves. Its high antioxidant and anti-inflammatory power is largely described in literature, such as to be indicated as the main molecule responsible for the healthy effects of EVOO. Recently, oleuropein has been tested for its capacity to regulate postprandial glycaemia in healthy individuals. Administered as such, the individuals subject to the trial have revealed a significant decrease in glycaemia, with stimulation for the release of insulin. Such studies enable oleuropein to be produced as a useful natural alternative to drugs for controlling glycaemia in healthy and diabetic subjects.

Trivalent chromium is an essential oligo-element known for its ability to regulate the homeostasis of glucose in animals. In particular, such micro-element represents the inorganic portion that activates an organic macromolecule known by the name of glucose tolerance factor (GTF). GTF, in turn, is responsible for the regulation of the activity of the enzymes appointed for the metabolism of glucose in animals. It is known from scientific literature that a trivalent chromium deficiency can cause severe alterations to the metabolism of sugars, until causing the classic symptoms of diabetic disease. An essential condition, for the purpose of the intestinal absorption of trivalent chromium, is its chemical form as an organic salt (chromium picolinate, nicotinate, ....). In fact, inorganic trivalent chromium is absorbed to an extent of no more than 3%, whereas its organic salt boasts a bioavailability even greater than 70%. To date, European Regulation No. 432/2012 attributes to trivalent chromium the health claim according to which such micro-element can contribute to the maintenance of normal levels of glucose in the blood.

Insulin resistance is defined as an insufficient response of the tissues to the effects of circulating insulin. The most important results of insulin resistance are a reduction in the intake of glucose by the skeletal muscles, reduced insulin-mediated inhibition of hepatic sugar production and reduced ability to inhibit lipolysis in the fatty tissues. Insulin resistance has been recognized as one of the main predictive factors for the development of various metabolic diseases, such as type 2 diabetes and metabolic syndrome. *In vitro* studies have demonstrated a significant relationship between the intake of carotenoids and the degree of insulin resistance. The results have revealed an inverse relationship between beta-carotene and lycopene concentration and the degree of glucose tolerance. This test implies that carotenoids can increase insulin sensitivity. Clinical trials demonstrate that low plasma levels of carotenoids are connected with greater insulin resistance in healthy volunteers. Thiazolidinediones are a class of anti-diabetic drugs, which are used in clinical practice for increasing insulin resistance, as PPARγ receptor agonists. It has been reported that bixin, a particular carotenoid, is able to increase insulin sensitivity through the expression of the PPARγ gene. The carotenoid astaxanthin has a role in reinforcing the activity of the metabolic enzymes appointed to have hypoglycaemic and antidiabetic effects. Similar properties have been described for fucoxanthin. The main source of carotenoids in the Mediterranean diet is represented by tomato. However, the concentration of such compounds is much higher in the skin than in the pulp. It is possible to reach 12 mg per 100 g of wet product, almost 5 times the concentration normally found in the pulp. This aspect demonstrates that tomato skins, as agri-food production waste, can represent an important source of carotenoids, which can be usefully recovered for nutraceutical applications for health purposes.

US2019/280799 A1 discloses compositions comprising ABA, which are useful in reducing glycaemia.

US2019/280799 A1 and Zocchi et al. (Frontiers in Nutrition 2017, 4, article 24) discloses that there are known fruits which are considered rich source of ABA: fig, apricot, bilberry, banana, apple, olive.

Guri et al (Clinical Nutrition 2007, 26,107-116) discloses that ABA could be used as a nutritional intervention against type II diabetes and obesity-related inflammation. The aim of the present invention is that of providing a nutraceutical composition that can at least be a useful alternative for the reduction of glycaemia or that can increase the already known effects of the aforesaid products.

### SUMMARY OF THE INVENTION

It has been surprisingly discovered that the main ABA content is displayed, with respect to immature fruits of different categories of fruits, in the immature fruits of hairless peach varieties. Therefore, the subject matter of the present patent application is a nutraceutical composition according to claim 1, useful for controlling glycaemia, preferably comprising freeze-dried immature fruits of the hairless peach varieties.

The nutraceutical composition according to the present invention is useful for reducing glycaemia.

The subject matter of the present invention is also a method for preparing freeze-dried immature fruit of the hairless peach varieties, said method comprising:
i) harvesting after flowering when they are 2-4 cm in diameter;
ii) freezing at a temperature in the range -50 to -80 ° C;
iii) freeze-drying;
iv) grinding.

For one aspect, the present invention relates to the use of immature fruits of the hairless peach varieties as a source of ABA.

### DETAILED DESCRIPTION OF THE INVENTION

The nutraceutical composition of the present invention also comprises oleuropein (preferably from olive leaf extract), trivalent chromium in form of an organic salt thereof and lycopene (preferably from freeze dried tomato skins).

It has been surprisingly discovered that the combination of these components produces a synergistic effect in the reduction of glycaemia.

Preferably, a composition according to the invention comprises:

| | |
|---|---|
| freeze-dried peach immature fruits | 45 - 46% |
| olive leaf extract | 8 - 9% |
| freeze-dried tomato skins | 45 - 46% |
| rivalent chromium | 0.5 - 1% |

where the percentages indicated are expressed in weight calculated with respect to the total weight of the composition.

The composition according to the invention is obtained by simply mixing the components in the required quantities with normal mixers.

The composition is normally formulated in powders, capsules, tablets, using known techniques (encapsulation, compression, controlled release, microgranules, nanocapsules, multilayers) as described in the pharmacopoeia for the preparation of pharmaceutical formulations for oral use.

A particular example of a pharmaceutical formulation according to the invention consists of sachets containing:
500-510 mg of freeze-dried immature peach, preferably 500 mg;
90-100 mg of olive leaf extract, preferably 100 mg;
500-510 mg of freeze-dried tomato skins, preferably 500 mg;
0.05-0.15 mg of trivalent chromium, preferably 0.05 mg.

In a particularly preferred way, the composition can contain:

| | |
|---|---|
| ABA | 7-10 mcg, preferably 7 mcg; |
| oleuropein | 15-25 mg, preferably 18 mg; |
| lycopene | 15-25, preferably 20 mg. |

A particular example of a pharmaceutical formulation according to the invention is a sachet comprising a composition consisting of:
freeze-dried immature peach, 500 mg; olive leaf extract, 100 mg; freeze-dried tomato skins, 500 mg; trivalent chromium, 0.05 mg. In particular, the composition preferably comprises: ABA, 7 mcg; oleuropein, 18 mg; lycopene, 20 mg.

The present invention can be better understood in the light of the following embodiment examples.

### EXPERIMENTAL PART

### EXAMPLE 1 - Study of the ABA content in immature fruits of different categories of fruits

The immature fruits from the thinning process are unripe fruits belonging to the following categories: apples, peaches, apricots. A study performed by our research group from the *NutraPharmaLabs* of the Pharmacy Department at the University of Naples "Federico II", on two consecutive years of thinning and harvesting immature fruits, highlighted the following ABA contents (Tab. 1):

| **Tab. 1. Abscisic acid (ABA) content in immature fruits** | | | | |
|---|---|---|---|---|
| | Peaches with hair | Hairless Peaches | Apples | Apricots |
| mcg/g fresh weight | 0.4 - 1.4 | 3.0 - 6.0 | 0.5 - 1.1 | 0.2 - 0.5 |
| mcg/g dry weight | 8.0 - 11.1 | 12.0 - 18.0 | 7.1 - 9.2 | 5.8 - 7.3 |

Surprisingly, as reported in table 1, it has been discovered that the highest ABA content is highlighted in the immature fruits of the varieties of peaches without hair. Therefore, the best candidate, for the purpose of the formulation of a useful nutraceutical for controlling glycaemia, is represented by the immature fruits of varieties of peaches without hair.

### EXAMPLE 2 - Preparation of the freeze-dried immature peach fruit

The immature peach fruits must be harvested when after flowering they measure 2-4 centimetres in diameter. Then, they are frozen at -50 -80 °C, freeze-dried and ground.

### EXAMPLE 3 - Preparation of olive leaf extract

The olive leaves were subjected to extraction with 96% ethanol at 30°C for 5 hours. The liquid thus collected is centrifuged, collecting the liquid part, which is then subjected to spray-drying in the presence of maltodextrin, giving the extract the form of a fine powder.

### EXAMPLE 4 - Preparation of the freeze-dried tomato skins

The tomatoes are ground and subjected to the separation of the pulp from the skins. The skins are collected, for freezing at -50/-80 °C, freeze-drying and grinding.

### EXAMPLE 5 - Preparation of sachets

A mixture of freeze-dried immature peach fruits (obtained as in example 2), freeze-dried tomato skins (obtained as in example 4) and olive leaf extract (obtained as in example 3) and trivalent chromium in the form of picolinate or nicotinate salt, in the suitable proportions, was prepared for filling the sachet so as to ensure a total content consisting of: freeze-dried immature peach fruits, 500 mg; olive leaf extract, 100 mg; freeze-dried tomato skins, 500 mg; trivalent chromium, 0.05 mg. In particular, the composition must comprise: ABA, 7 mcg; oleuropein, 18 mg; lycopene, 20 mg.

### EXAMPLE 6 - Clinical data

The efficacy of a powder formulation (based on freeze-dried immature peach fruits, 500 mg; olive leaf extract, 100 mg; freeze-dried tomato skins, 500 mg; trivalent chromium, 0.05 mg; in particular, the composition comprised ABA, 7 mcg; oleuropein, 18 mg; lycopene, 20 mg) was evaluated on the glycaemic peak reached by healthy individuals subjected to glucose load curve. By definition, it is said that an individual is subjected to a glucose load curve when he/she takes an aqueous solution containing 75 g of glucose, to then monitor the glycaemia every 15 min for about 120 min, in order to evaluate the glycaemic response. A monocentric randomized, placebo-controlled, parallel group trial was conducted on 60 healthy volunteers, 25 men and 35 women, aged between 25 and 70, of white race. The subjects were subjected to the test after fasting for at least 14 hours. The exclusion criteria were: smoking, obesity (BMI >30 kg/m²), diabetes, liver disease, kidney disease, heart disease, hereditary chronic diseases, taking pharmaceuticals and supplements for hyperglycaemia, taking pharmaceuticals and supplements based on ABA, olive polyphenols, carotenoids, intense physical exercise (>10 h/week), pregnant women, women suspected of being pregnant, woman with planned pregnancy, breastfeeding women, pollen allergy. The subjects received a written and oral information notice and the trial protocol before providing their written consent. The individuals were randomized in 6 groups. The participants of each group performed two glucose load curves, one to evaluate the glycaemic response under baseline conditions, and a subsequent one two days later, simultaneously taking the sugar solution and as follows:
group 1, placebo;
group 2, freeze-dried immature peach, 500 mg;
group 3, olive leaf extract, 100 mg;
group 4, freeze-dried tomato skins, 500 mg;
group 5, trivalent chromium, 0.05 mg;
group 6, mix comprised of freeze-dried immature peach, 500 mg; olive leaf extract, 100 mg; freeze-dried tomato skins, 500 mg; trivalent chromium, 0.05 mg.

The results obtained were as follows:

| **Tab. 2. Monitoring of the glycaemic peak (mg/dL) at 30 min during the glucose load curve.** | | | |
|---|---|---|---|
| Treatment | Pre-treatment | Post-treatment | **Variation (%)** |
| **Placebo** | 151.4 ± 6.2 | 150.0 ± 5.9 | **-1.0** |
| **Freeze-dried immature peach** | 147.4 ± 5.8 | 140.6 ± 6.1 | **-4.6** |
| **Olive leaf extract** | 150.3 ± 7.1 | 143.1 ± 6.3 | **-4.8** |
| **Freeze-dried tomato skins** | 148.5 ± 7.3 | 142.0 ± 5.1 | **-4.4** |
| **Trivalent chromium** | 152.1 ± 7.4 | 148.3 ± 5.2 | **-2.5** |
| **Mix** | 149.2 ± 6.8 | 113.7 ± 4.8 | **-23.8** |

| | | | |
|---|---|---|---|
| The values are expressed as mean ± SD (n = 5). The results were significantly different at a level P = 0.001. | | | |

As it is possible to notice, the formulation defined as mix, comprising freeze-dried immature peach, olive leaf extract, freeze-dried tomato skins, and trivalent chromium, promoted the greatest decrease in the glycaemic peak, greater than the sum of the effects of the components administered individually. Such formulation clearly indicates a synergistic effect between the various constituents of the formulation.

The results obtained indicate the possibility of an innovative treatment that can represent a valid alternative in clinical practice.

## Claims

1. A nutraceutical composition, comprising abscisic acid (ABA), oleuropein, trivalent chromium in the form of organic salt thereof, and lycopene.

2. A composition according to claim 1 in which ABA is present as freeze-dried immature fruits of the hairless peach varieties, oleuropein is present as an olive leaf extract, trivalent chromium is present in the form of picolinate or nicotinate and lycopene is present as freeze-dried tomato skins.

3. A composition according to claim 2 comprising:
| | |
|---|---|
| freeze-dried peach immature fruits | 45 - 46% |
| olive leaf extract | 8 - 9% |
| freeze-dried tomato skins | 45 - 46% |
| trivalent chromium | 0.5 - 1% |
where the percentages indicated are expressed in weight calculated with respect to the total weight of the composition.

4. A composition according to any one of claims 1-3 formulated in powders, capsules or tablets.

5. A composition according to claim 4 in the form of sachets containing:
500-510 mg of freeze-dried immature peach, preferably 500 mg;
90-100 mg of olive leaf extract, preferably 100 mg;
500-510 mg of freeze-dried tomato skins, preferably 500 mg;
0.05-0.15 mg of trivalent chromium, preferably 0.05 mg.

6. A composition according to any one of claims 1-5 containing:
| | |
|---|---|
| ABA | 7-10 mcg; |
| oleuropein | 15-25 mg; |
| lycopene | 15-25 mg |
| trivalent chromium | 0.05-0.15 mg. |

7. A composition according to any one of claims 1-6 for use in reducing blood glucose.

8. A method for the preparation of a freeze-dried immature peach of the hairless peach varieties, said method comprising
i) harvesting after flowering when they are 2-4 cm in diameter;
ii) freezing at a temperature in the range -50 to -80 ° C;
iii) freeze-drying;
iv) grinding.

9. Use of immature fruits of hairless peach varieties as a source of ABA.

## Patentansprüche

1. Nutrazeutische Zusammensetzung, umfassend Abscisinsäure (ABA), Oleuropein, dreiwertiges Chrom in Form eines organischen Salzes davon und Lycopin.

2. Zusammensetzung nach Anspruch 1, in der ABA als gefriergetrocknete, unreife Früchte der haarlosen Pfirsichsorten vorliegt, Oleuropein als Olivenblattextrakt vorliegt, dreiwertiges Chrom in Form von Picolinat oder Nicotinat vorliegt und Lycopin als gefriergetrocknete Tomatenhaut vorliegt.

3. Zusammensetzung nach Anspruch 2, umfassend:
| | |
|---|---|
| gefriergetrocknete, unreife Pfirsichfrüchte | 45-46 % |
| Olivenblattextrakt | 8-9 % |
| gefriergetrocknete Tomatenhaut | 45-46 % |
| dreiwertiges Chrom | 0,5-1 % |
wobei die angegebenen Prozentwerte in Gewicht ausgedrückt sind und bezogen auf das Gesamtgewicht der Zusammensetzung berechnet sind.

4. Zusammensetzung nach einem der Ansprüche 1-3, die als Pulver, Kapseln oder Tabletten formuliert ist.

5. Zusammensetzung nach Anspruch 4 in Form von Portionsbeuteln, enthaltend:
500-510 mg gefriergetrockneten, unreifen Pfirsich, vorzugsweise 500 mg;
90-100 mg Olivenblattextrakt, vorzugsweise 100 mg;
500-510 mg gefriergetrocknete Tomatenhaut, vorzugsweise 500 mg;
0,05-0,15 mg dreiwertiges Chrom, vorzugsweise 0,05 mg.

6. Zusammensetzung nach einem der Ansprüche 1-5, enthaltend:
| | |
|---|---|
| ABA | 7-10 mcg; |
| Oleuropein | 15-25 mg; |
| Lycopin | 15-25 mg |
| dreiwertiges Chrom | 0,05-0,15 mg. |

7. Zusammensetzung nach einem der Ansprüche 1-6 zur Verwendung bei der Reduzierung des Blutzuckers.

8. Verfahren zur Herstellung eines gefriergetrockneten, unreifen Pfirsichs der haarlosen Pfirsichsorten, wobei das Verfahren umfasst
i) Ernten nach der Blüte, wenn sie einen Durchmesser von 2-4 cm aufweisen;
ii) Einfrieren bei einer Temperatur im Bereich von -50 bis -80 °C;
iii) Gefriertrocknen;
iv) Mahlen.

9. Verwendung von unreifen Früchten von haarlosen Pfirsichsorten als Quelle von ABA.

## Revendications

1. Composition nutraceutique, comprenant de l'acide abscissique (ABA), de l'oleuropéine, du chrome trivalent sous forme de sel organique de celui-ci et du lycopène.

2. Composition selon la revendication 1, dans laquelle l'ABA est présent sous forme de fruits immatures lyophilisés des variétés de pêches glabres, l'oleuropéine est présente sous forme d'extrait de feuille d'olivier, le chrome trivalent est présent sous forme de picolinate ou de nicotinate et le lycopène est présent sous forme de peaux de tomates lyophilisées.

3. Composition selon la revendication 2 comprenant :
| | |
|---|---|
| fruits immatures de pêche lyophilisés | 45 à 46% |
| extrait de feuilles d'olivier | 8 à 9% |
| peaux de tomates lyophilisées | 45 à 46% |
| chrome trivalent | 0,5 à 1% |
où les pourcentages indiqués sont exprimés en poids calculé par rapport au poids total de la composition.

4. Composition selon l'une quelconque des revendications 1 à 3, formulée en poudres, capsules ou comprimés.

5. Composition selon la revendication 4 sous forme de sachets, contenant :
500 à 510 mg de pêche immature lyophilisée, de préférence 500 mg ;
90 à 100 mg d'extrait de feuille d'olivier, de préférence 100 mg ;
500 à 510 mg de peaux de tomates lyophilisées, de préférence 500 mg ;
0,05 à 0,15 mg de chrome trivalent, de préférence 0,05 mg.

6. Composition selon l'une quelconque des revendications 1 à 5, contenant :
| | |
|---|---|
| ABA | 7 à 10 mcg ; |
| oleuropéine | 15 à 25 mg ; |
| lycopène | 15 à 25 mg |
| chrome trivalent | 0,05 à 0,15 mg. |

7. Composition selon l'une quelconque des revendications 1 à 6, destinée à être utilisée pour réduire la glycémie.

8. Procédé pour la préparation d'une pêche immature lyophilisée des variétés de pêches glabres, ledit procédé comprenant
i) la récolte après la floraison lorsqu'elles ont un diamètre de 2 à 4 cm ;
ii) la congélation à une température comprise entre -50 et -80 °C ;
iii) la lyophilisation ;
iv) le broyage.

9. Utilisation de fruits immatures des variétés de pêches glabres comme source d'ABA.
